(19) Europäisches Patentamt
European Patent Office
Office européen des brevets

(11) Veröffentlichungsnummer: **0 008 565**
**A1**

# (12) EUROPÄISCHE PATENTANMELDUNG

(21) Anmeldenummer: 79810073.1

(22) Anmeldetag: 22.08.79

(51) Int. Cl.[3]: C 07 D 233/22
A 01 N 43/50
//A61K31/415

(30) Priorität: 28.08.78 CH 9079/78
10.08.79 CH 7370/79

(43) Veröffentlichungstag der Anmeldung: 05.03.80 Patentblatt 80/5

(84) Benannte Vertragsstaaten: AT BE CH DE FR GB IT NL

(71) Anmelder: CIBA-GEIGY AG
Patentabteilung Postfach
CH-4002 Basel(CH)

(72) Erfinder: Böger, Manfred
Lindenstrasse 33
D-7858 Weil am Rhein 5(DE)

(72) Erfinder: Drabek, Jozef, Dr.
Benkenstrasse 12
CH-4104 Oberwil(CH)

(72) Erfinder: Mattern, Günter, Dr.
Sichternstrasse 35
CH-4410 Liestal(CH)

(72) Erfinder: Traber, Walter, Dr.
Neueneichweg 12
CH-4153 Reinach(CH)

(54) **Bis-(phenoxy-alkyl-2-imidazolin)-1,1'-sulfide, Verfahren zu ihrer Herstellung, Mittel welche diese Sulfide als aktive Komponente enthalten und deren Verwendung zur Bekämpfung von Schädlingen.**

(57) Verbindung der Formel I

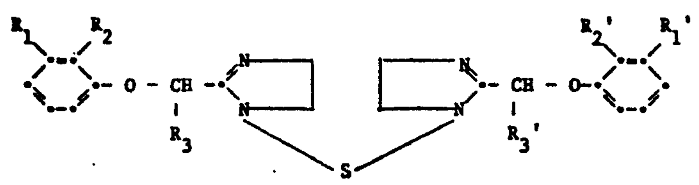

worin $R_1$, $R_1'$, $R_2$ und $R_2'$ je Chlor oder Methyl bedeuten und $R_3$ und $R_3'$ je Wasserstoff oder $C_1$-$C_4$-Alkyl bedeuten mit pestizider insbesondere akarizider Wirkung.

EP 0 008 565 A1

0008565



CIBA-GEIGY AG
Basel (Schweiz)

5-11996/1+2

Bis-(phenoxy-alkyl-2-imidazolin)-1,1'-sulfide, Verfahren zu ihrer Herstellung, Mittel welche diese Sulfide als aktive Komponente enthalten und deren Verwendung zur Bekämpfung von Schädlingen.

Die vorliegende Erfindung betrifft neue Bis-(phenoxy-alkyl-2-imidazolin)-1,1'-sulfide, welche eine Wirkung gegen Schädlinge besitzen, und Verfahren zu ihrer Herstellung sowie Schädlingsbekämpfungsmittel, welche die vorstehend genannten Sulfide als aktive Komponente enthalten, und Verfahren zur Bekämpfung von Schädlingen unter Verwendung der neuen Verbindungen.

2-(Phenoxy-alkyl)-2-imidazolinderivate mit pestizider vor allem ektoparasitizider Wirkung sind bekannt (siehe z.B. südafrikanische Patentanmeldung Nr. 78/2449, japanische Patentveröffentlichung 76/106739, DOS 2,756,638 und DOS 2,756,639). Nach vorliegender Erfindung werden neuartige Verbindungen dieses Typs bereitgestellt, die ebenfalls eine Wirkung gegen Schädlinge, insbesondere gegen Vertreter der Ordnung Akarina aufweisen und welche aufgrund ihrer vorteilhaften biologischen Eigenschaften für die praktische Anwendung besonders geeignet sind.

Die erfindungsgemässen, neuen Bis-(phenoxy-alkyl-2-imidazolin)-1,1'-sulfide entsprechen der Formel I

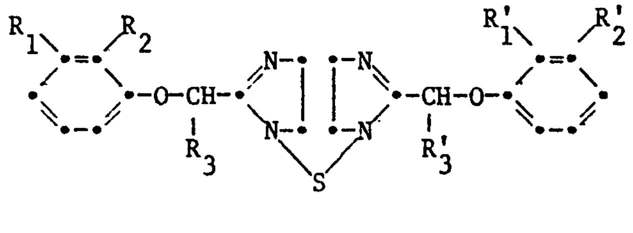

(I)

worin $R_1$, $R_1'$, $R_2$ und $R_2'$ unabhängig voneinander ein Chloratom oder eine Methylgruppe und $R_3$ und $R_3'$ unabhängig voneinander ein Wasserstoffatom

oder eine $C_1$-$C_4$-Alkylgruppe bedeuten.

Die für $R_3$ und $R_3'$ in Frage kommenden Alkylgruppen sind die Methyl-, Aethyl-, n-Propyl-, i-Propyl-, n-Butyl-, i-Butyl-, s-Butyl- und t-Butylgruppe.

In den Verbindungen der Formel I werden die folgenden Substituententypen sowie Kombinationen derselben untereinander bevorzugt:

1) Bei $R_1$, $R_1'$, $R_2$ und $R_2'$ : gleichzeitig Methyl oder Chlor;
2) Bei $R_3$ und $R_3'$: gleichzeitig Wasserstoff oder Aethyl, vor allem Aethyl.

Die Verbindungen der Formel I liegen in Form von Säureadditionssalzen z.B. Mineral-Salzen vor und können erfindungsgemäss in Form ihrer Salze verwendet werden. Unter dem Begriff der vorliegenden Erfindung versteht man demzufolge sowohl die freien Verbindungen der Formel I als auch deren Säureadditionssalze.

Es hat sich nun überraschenderweise gezeigt, dass die erfindungsgemässen Verbindungen der Formel I eine wertvolle Wirkung sowohl gegen pflanzenschädigende Akariden (Milben: z.B der Familien Tetranychidae, Tarsonemidae, Eriophydae, und Glycyphagidae) als auch gegen ektoparasitäre Akariden (Milben und Zecken: z.B. der Familien Ixodidae, Argasidae, Sarcoptidae und Dermanyssidae), welche an Nutztieren Schaden anrichten, aufweisen. Darüber hinaus wurde weiter festgestellt, dass diese akariziden Eigenschaften mit einer für die praktische Anwendung günstigen Toxizität gegenüber Warmblütern gekoppelt sind, wobei sich die Verbindungen der Formel I sowie deren für Warmblüter nicht-toxische Säureadditionssalze zur Bekämpfung von Schädlingen der Ordnung Akarina in Kulturen von Nutz- und Zierpflanzen, hauptsächlich auf dem Gebiet des Obst- bzw. Citrusbaues, sowie zur Bekämpfung von ektoparasitären Zecken und Milben bei Nutztieren besonders eignen.

Die Verbindungen der Formel I werden analog bekannten Verfahren hergestellt, in dem man z.B. eine Verbindung der Formel II

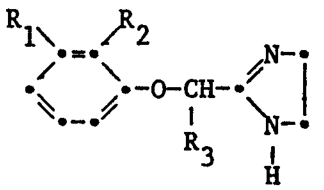

(II)

in Gegenwart einer Base mit einer Verbindung der Formel III

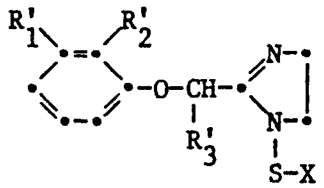

(III)

umsetzt, wobei in den Formeln II und III $R_1$, $R_1'$, $R_2$, $R_2'$, $R_3$ und $R_3'$ die unter Formel I bereits angegebenen Bedeutungen haben und X ein Halogenatom, insbesondere ein Chlor- oder Bromatom bedeutet.

Das Verfahren wird zweckmässig bei einer Temperatur zwischen -20° und 30°C, bei normalem oder leicht erhöhtem Druck und vorzugsweise in Gegenwart eines gegenüber den Reaktionsteilnehmern inerten Lösungs- oder Verdünnungsmittel durchgeführt.

Als Lösungs- oder Verdünnungsmittel eignen sich z.B. Aether und ätherartige Verbindungen wie Di-äthyläther, Di-isopropyläther, Dioxan und Tetrahydrofuran; aromatische Kohlenwasserstoffe wie Benzol, Toluol und Xylole; und Ketone wie Aceton, Methyläthylketon und Cyclohexanon.

Als für dieses Verfahren geeignete Basen kommen insbesondere tertiäre Amine, wie Trialkylamine, Pyridine und Dialkylaniline ferner Hydroxide, Oxide, Carbonate und Bicarbonate von Alkali- und Erdalkalimetallen sowie Alkalimetallalkoholate wie z.B. Kalium-tert.butylat und Natriummethylat in Betracht.

Die auf diese Weise hergestellten Verbindungen der Formel I können nach an sich bekannten Methoden in ihre Säuresalze überführt werden.

Die Verbindungen der Formel I worin $R_3$ und/oder $R_3'$ eine Alkylgruppe bedeutet, liegen in Form von optisch aktiven Isomeren vor. Wenn deshalb bei der Herstellung keine optisch aktiven Ausgangsmaterialien verwendet werden, gelangt man zwangsläufig zu racemischen Gemischen. Solche Isomerengemische können z.B. mit Hilfe chromatographischer Trennmethoden in die einzelnen isomeren Formen aufgetrennt werden. Unter dem Begriff der gegenwärtigen Erfindung versteht man sowohl die einzelnen optisch aktive Isomere, als auch deren Gemische.

Die in dem vorstehend aufgeführten Verfahren verwendeten Ausgangsstoffe sind bekannt (vgl. Südafrikanische Patentanmeldung Nr. 76/2449 und DOS 2,756,638) bzw. können analog den bekannten Methoden hergestellt werden.

Die Verbindungen der Formel I werden erfindungsgemäss als solche verwendet oder bilden einen Bestandteil von Mitteln, welche noch geeignete Trägerstoffe oder Zuschlagstoffe oder Gemische solcher Stoffe enthalten.

Geeignete Träger- und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die akarizide Wirkung der erfindungsgemässen Mittel lässt sich durch Zusatz anderer Akarizide und/oder Insektizide wesentlich verbreitern. Als Zusätze eignen sich z.B.: org. Phosphorverbindungen; Nitrophenole und deren Derivate; Formamidine; Harnstoffe; pyrethinartige Verbindungen; Karbamate und chlorierte Kohlenwasserstoffe.

Die erfindungsgemässen Mittel können z.B. als Stäubemittel, Granulate, Dispersionen, Lösungen und Aufschlämmungen sowie als in Wasser dispergierbare Spritzpulver, Pasten, Emulsionen und Emulsionskonzentraten vorliegen und angewendet werden. Der Gehalt an Wirkstoff (Verbindung der Formel I) in den oben beschriebenen Mitteln liegt zwischen 0,1 bis 95%, dabei ist zu erwähnen, dass bei der Applikation aus dem Flugzeug oder mittels anderer geeigneter Applikationsgeräte auch höhere Konzentrationen eingesetzt werden können.

Die Wirkstoffe der Formel I können beispielsweise wie folgt formuliert werden:

Emulsionskonzentrat I

| | |
|---|---|
| 20 Gew.-Teile | des oben genannten Wirkstoffes werden in |
| 70 Gew.-Teilen | Xylol gelöst und mit |
| 10 Gew.-Teilen | eines Emulgiermittels, bestehend aus einem Gemisch eines arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion. |

Emulsionskonzentrat II

| | |
|---|---|
| 5 bis max 30 Gew.-Teile | Wirkstoff werden unter Rühren bei Zimmertemperatur in |
| 30 Gew.-Teilen | Dibutylphthalat |
| 10 Gew.-Teilen | Solvent 200 (niederviskoses hocharomatisches Erdöldestillat) |
| 15 bis 35 Gew.-Teilen | Dutrex 238 FC (viskoses hocharomatisches Erdöldestillat) gelöst und mit |
| 10 Gew.-Teilen | eines Emulgatorgemisches, bestehend aus Ricinusöl-polyglykoläther und dem Calciumsalz der Dodecylbenzolsulfonsäure, versetzt. Das so erhaltene Emulsionskonzentrat gibt in Wasser milchige Emulsionen. |

Spritzpulver

| | |
|---|---|
| 5 bis 30 Gew.-Teile | des Wirkstoffes werden in einer Mischapparatur mit |
| 5 Gew.-Teilen | eines aufsaugenden Trägermaterials (Kieselsäure K 320 oder Wessalon S) und |
| 55 bis 80 Gew.-Teilen | eines Trägermaterials (Bolus alba oder Kaolin B 24) und einem Dispergiermittelgemisch, bestehend aus |
| 5 Gew.-Teilen | eines Na-lauryl-sulfonates und |
| 5 Gew.-Teilen | eines Alkyl-aryl-polyglykoläthers, intensiv vermischt. Diese Mischung wird auf einer Stift- oder Luftstrahlmühle bis auf 5-15 µm gemahlen. Das so erhaltene Spritzpulver gibt in Wasser eine gute Suspension. |

Stäubemittel

| | |
|---|---|
| 5 Gew.-Teile | feingemahlener Wirkstoff werden mit |
| 2 Gew.-Teilen | einer gefällten Kieselsäure und |
| 93 Gew.-Teilen | Talk intensiv gemischt. |

Pour-on-Lösung

| | | |
|---|---|---|
| Wirksubstanz | 30,0 g | |
| Natrium-dioctylsulfosuccinat | 3,0 g | |
| Benzylalkohol | 48,0 g | |
| Erdnussöl | 19,8 g | |
| | 100,8 g | = 100 ml |

Die Wirksubstanz wird in dem Benzylalkohol unter Rühren, eventuell auch unter leichtem Erwärmen, gelöst. Zu der Lösung wird das Natrium-dioctylsulfosuccinat und das Erdnussöl gegeben und unter Erwärmen und gründlichem Durchmischen gelöst.

Die folgenden Beispiele dienen zur näheren Erläuterung der Erfindung:

Beispiel 1: Herstellung von Bis-((2,3-dimethylphenoxymethyl)-2-imidazolin)-1,1'-sulfid.

Zu einer Lösung von 20,4 g 2-(2,3-Dimethylphenoxymethyl)-2-imidazolin in 200 ml Methylenchlorid wurden bei 0°C 11 g Triäthylamin zugegeben und anschliessend 5,15 g frisch destilliertes Schwefelchlorid langsam zugetropft. Das Reaktionsgemisch wurde anschliessend ca. 2 Stunden im Eisbad nachgerührt, die erhaltene Suspension mit Wasser versetzt und die organische Phase mehrmals mit Wasser gewaschen. Nach dem Trocknen über Natriumsulfat wurde die organische Phase völlig eingedampft und mit Hexan verrührt. Nach Umkristallisierung der erhaltenen festen Ausfällung aus Toluol/Hexan erhielt man das Bis-(2,3-dimethylphenoxymethyl)-2-imidazolin)-1,1'-sulfid (Verbindung Nr. 1) der Formel

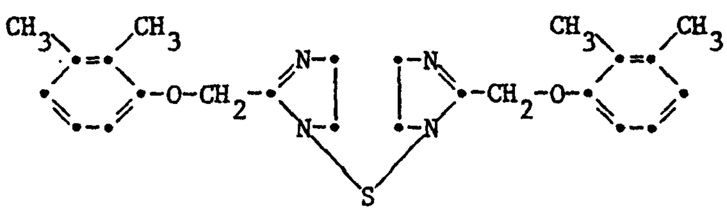

als ein hellbeiges Pulver mit einem Smp. von 112-112°C. Analog zu den vorstehend beschriebenen Herstellungsverfahren können auch die folgenden Verbindungen der Formel I hergestellt werden

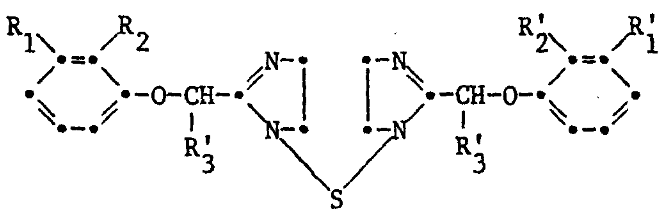

| Verb. Nr. | $R_1$ | $R_2$ | $R_3$ | $R'_1$ | $R'_2$ | $R'_3$ | Physikalische Daten |
|---|---|---|---|---|---|---|---|
| 2 | $CH_3$ | $CH_3$ | $C_2H_5$ | $CH_3$ | $CH_3$ | $C_2H_5$ | Smp. 84-86°C |
| 3 | $CH_3$ | $CH_3$ | $CH_3$ | $CH_3$ | $CH_3$ | $CH_3$ | |
| 4 | $CH_3$ | $CH_3$ | n-$C_4H_9$ | $CH_3$ | $CH_3$ | n-$C_4H_9$ | |
| 5 | Cl | Cl | H | Cl | Cl | H | |
| 6 | Cl | Cl | $C_2H_5$ | Cl | Cl | $C_2H_5$ | |

Beispiel 2: Wirkung gegen pflanzenschädigende Akariden (Milben): Tetranychus urticae (OP-sensible) und Tetranychus cinnabarinus (OP-tolerant)

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sen.) oder Tetranychus cinnabarinus (OP-tol.) belegt. (Die Toleranz bezieht sich auf die Verträglichkeit von Diazinon).

Die so behandelten infestierten Pflanzen wurden mit einer Versuchslösung enthaltend 400 oder 200 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25°C.

Verbindungen der Formel I zeigen in diesem Versuch eine positive Wirkung gegen Individuen der Spezies Tetranychus urticae und Tetranychus cinnabarius.

Beispiel 3: Wirkung gegen ektoparasitäre Akariden (Zecken: Rhipicephalus bursa (Imagines und Larven), Amblyomma hebraeum (♀ Imagines, Nymphen und Larven) und Boophilus microplus (Larven O.P. sensible und OP-tolerant).

Als Testobjekte wurden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere wurden für kurze Zeit in eine wässrige Emulsion bzw. Lösung enthaltend 0,1; 1,0; 10; 50 oder 100 ppm der zu prüfenden Verbindung getaucht.

Die in Teströhrchen befindlichen Emulsionen bzw. Lösungen wurden dann mit Watte aufgenommen und die benetzten Testtiere in den so kontaminierten Röhrchen belassen. Eine Auswertung der erzielten Abtötungsrate bei jeder Konzentration erfolgte für Larven nach 3 Tagen und für Nymphen und Imagines nach 14 Tagen.

Verbindungen der Formel I zeigen in diesem Versuch eine gute Wirkung gegen Larven, Nymphen und Imagines oder Spezies Rhipicephalus bursa und Amblyomma hebraeum sowie gegen Larven (OP-res. und OP-sens.) der Spezies Boophilus microplus.

Patentansprüche

(für alle benannten Länder ausser Oesterreich)

1. Eine Verbindung der Formel I

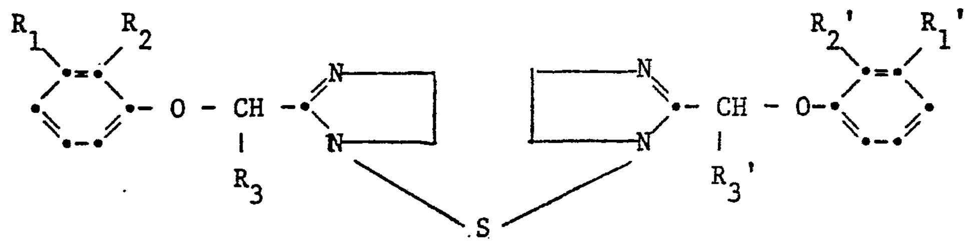

(I)

worin $R_1$, $R_1'$, $R_2$ und $R_2'$ unabhängig voneinander ein Chloratom oder eine Methylgruppe und $R_3$ und $R_3'$ unabhängig voneinander ein Wasserstoffatom oder eine $C_1$-$C_4$-Alkylgruppe bedeuten, und ihrer Säureadditionssalze.

2. Verbindung nach Anspruch 1 der Formel I, worin $R_1$, $R_1'$, $R_2$ und $R_2'$ gleichzeitig ein Chloratom oder eine Methylgruppe bedeuten.

3. Verbindung nach Anspruch 1 oder 2 der Formel I, worin $R_3$ und $R_3'$ gleichzeitig ein Wasserstoffatom oder eine Aethylgruppe bedeuten.

4. Verbindung nach Anspruch 3 der Formel I, worin $R_3$ und $R_3'$ gleichzeitig eine Aethylgruppe bedeuten.

5. Verbindung nach Anspruch 4 der Formel

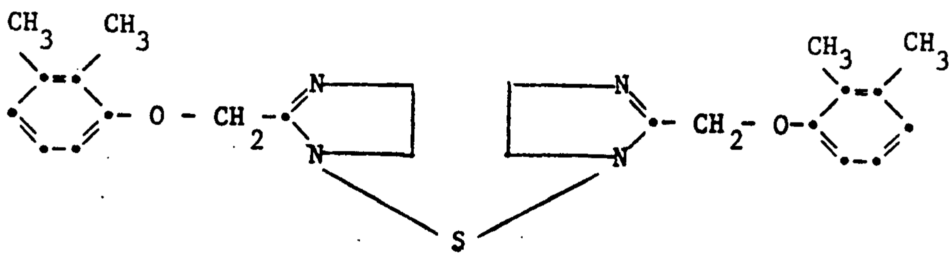

6. Verbindung nach Anspruch 4 der Formel

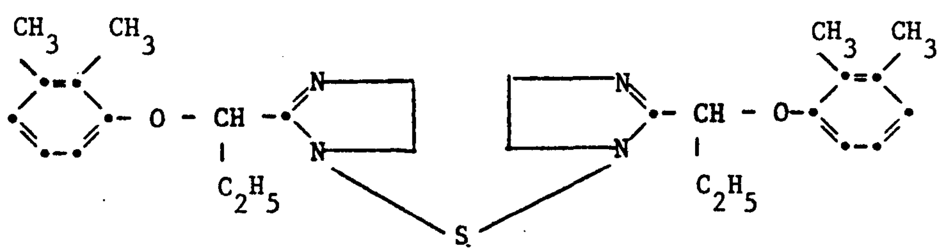

7. Verfahren zur Herstellung von in einem der Ansprüche 1 bis 6 definierten Verbindungen, dadurch gekennzeichnet, dass man eine Verbindung der Formel II

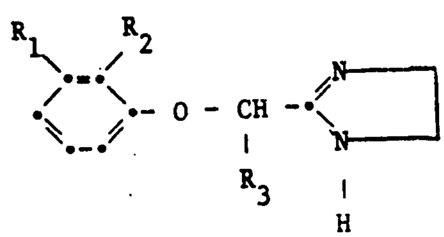

(II)

in Gegenwart einer Base mit einer Verbindung der Formel III

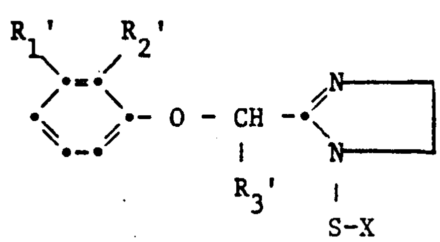

(III)

umsetzt, worin $R_1$, $R_1'$, $R_2$, $R_2'$, $R_3$ und $R_3'$ die entsprechenden, in einem der Ansprüche 1 bis 6 angegebenen Bedeutungen haben und X ein Halogenatom bedeutet.

8. Schädlingsbekämpfungsmittel enthaltend als aktive Komponente eine der in einem der Ansprüche 1 bis 6 definierten Verbindungen.

9. Verwendung von einer der in einem der Ansprüche 1 bis 6 definierten Verbindungen zur Bekämpfung von Vertretern der Ordnung Akarina.

10. Verwendung nach Anspruch 9 zur Bekämpfung von pflanzenschädigenden Vertretern der genannten Ordnung in Kulturen von Zier- oder Nutzpflanzen.

0008565



Patentansprüche

(für Oesterreich)

1. Schädlingsbekämpfungsmittel enthaltend als aktive Komponente eine Verbindung der Formel I

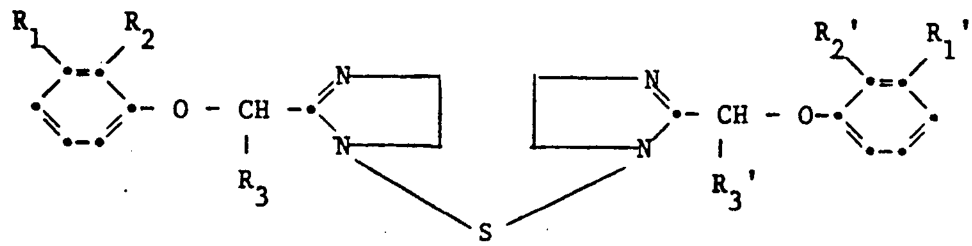

(I)

worin $R_1$, $R_1'$, $R_2$ und $R_2'$ unabhängig voneiander ein Chloratom oder eine Methylgruppe und $R_3$ und $R_3'$ unabhängig voneinander ein Wasserstoffatom oder eine $C_1$-$C_4$-Alkylgruppe bedeuten, oder eines ihrer Säureaditionssalze.

2. Mittel nach Anspruch 1 enthaltend als aktive Komponente eine Verbindung der Formel I, worin $R_1$, $R_1'$, $R_2$ und $R_2'$ gleichzeitig ein Chloratom oder eine Methylgruppe bedeuten.

3. Mittel nach Anspruch 1 oder 2 enthaltend als aktive Komponente eine Verbindung der Formel I, worin $R_3$ und $R_3'$ gleichzeitig ein Wasserstoffatom oder eine Aethylgruppe bedeuten.

4. Mittel nach Anspruch 3 enthaltend als aktive Komponente eine Verbindung der Formel I, worin $R_3$ und $R_3'$ gleichzeitig eine Aethylgruppe bedeuten.

5. Mittel nach Anspruch 4 enthaltend als aktive Komponente eine Verbindung der Formel

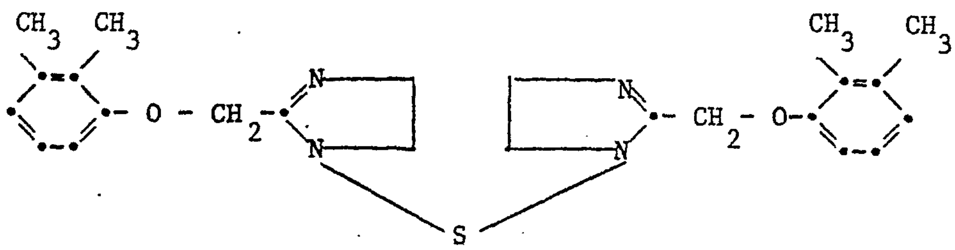

6. Mittel nach Anspruch 4 enthaltend als aktive Komponente eine Verbindung der Formel

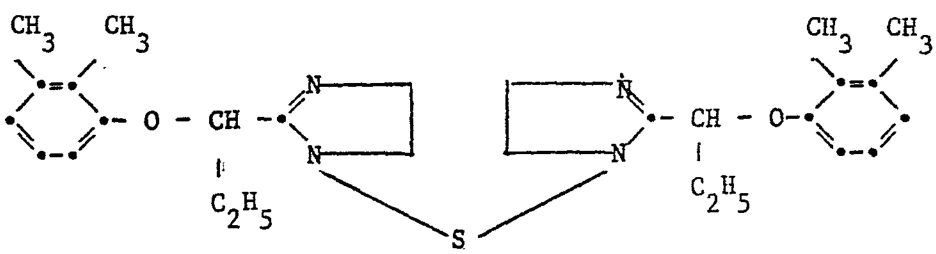

7. Verfahren zur Herstellung von in einem der Ansprüche 1 bis 6 definierten Verbindungen, dadurch gekennzeichnet, dass man eine Verbindung der Formel II

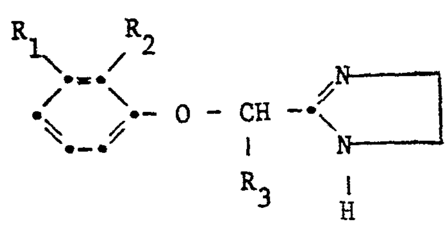

(II)

in Gegenwart einer Base mit einer Verbindung der Formel III

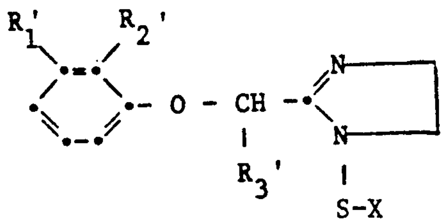

(III)

umsetzt, worin $R_1$, $R_1'$, $R_2$, $R_2'$, $R_3$ und $R_3'$ die entsprechenden in einem der Ansprüche 1 bis 6 angegebenen Bedeutungen haben und X ein Halogenatom bedeutet.

8. Verwendung von einer der in einem der Ansprüche 1 bis 6 definierten Verbindungen zur Bekämpfung von Vertretern der Ordnung Akarina.

9. Verwendung nach Anspruch 8 zur Bekämpfung von pflanzenschädigenden Vertretern der genannten Ordnung in Kulturen von Zier- oder Nutzpflanzen.

0008565

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 79 81 0073

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 1 935 479 (NORDMARK-WERKE)<br>* Seiten 9 und 10 *<br>-- | 1,8 |
| P | DE - A - 2 818 367 (CIBA-GEIGY)<br>* Seiten 4-9 *<br>---- | 1,8 |

KLASSIFIKATION DER ANMELDUNG (Int. Cl.³)

C 07 D 233/22
A 01 N 43/50//
A 61 K 31/415

RECHERCHIERTE SACHGEBIETE (Int. Cl.³)

C 07 D 233/22
A 01 N 43/50
A 61 K 31/415

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8,10

Unvollständig recherchierte Patentansprüche:

Nicht recherchierte Patentansprüche: 9

Grund für die Beschränkung der Recherche: Verfahren zur chirurgischen oder therapeutischen Behandlung des menschlichen oder tierischen Körpers. (Siehe Art. 52(4) des Europäischen Patentübereinkommens)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 23-11-1979 | DE BUYSER |

EPA Form 1505.1 06.78